# FASCICULE DE BREVET EUROPEEN

(11) **EP 1 189 932 B1**
(45) Date de publication et mention de la délivrance du brevet: **21.05.2003**
(21) Numéro de dépôt: 00940456.7
(22) Date de dépôt: 08.06.2000
(51) Int. Cl.: C07K 7/56, A61K 38/12, A61P 31/10

(54) **DERIVES DE L'ECHINOCANDINE, LEUR PROCEDE DE PREPARATION ET LEUR APPLICATION COMME ANTIFONGIQUES**
ECHINOCANDINDERIVATE, DEREN HERSTELLUNG UND VERWENDUNG ALS FUNGIZID
NOVEL ECHINOCANDIN DERIVATIVES, METHOD FOR PREPARING THE SAME AND USE AS ANTIFUNGAL AGENTS

(30) Priorité: 09.06.1999 FR 9907252
(43) Date de publication de la demande: 27.03.2002
(73) Titulaire: Aventis Pharma S.A., 92160 Antony (FR)
(72) Inventeur: CORBIER, Alain, F-91370 Verrières le Buisson (FR); FAUVEAU, Patrick, F-93190 Livry Gargan (FR); PIETRE-DISCHAMP, Nathalie, F-94490 Ormesson sur Marne (FR); SCHIO, Laurent, F-93140 Bondy (FR); VICAT, Pascale, F-75017 Paris (FR)
(74) Mandataire: Rousseau, Pierrick Edouard
(86) Numéro de dépôt international: FR0001569
(87) Numéro de publication internationale: WO00075178

(56) Documents cités:
- EP-A- 0 644 199
- EP-A- 0 736 541
- WO-A-96/13272
- WO-A-98/23637
- WO-A-99/29716

## Description

La présente invention concerne de nouveaux dérivés de l'échinocandine, leur procédé de préparation et leur application comme antifongiques.

L'utilisation de composés cyclohexapeptidiques comme agents antifongiques a déjà été décrite dans l'art antérieur, comme par exemple dans les documents EP-A-0 736 541 et WO 96/13 272-A.

Certains composés cyclohexapeptidiques peuvent également être utilisés comme agents antimicrobiens comme le divulguent par exemple les documents WO 98/23 637-A et EP-A-0 644 199.

L'invention a pour objet sous toutes les formes d'isomères possibles ainsi que leurs mélanges, les composés de formule (I) : 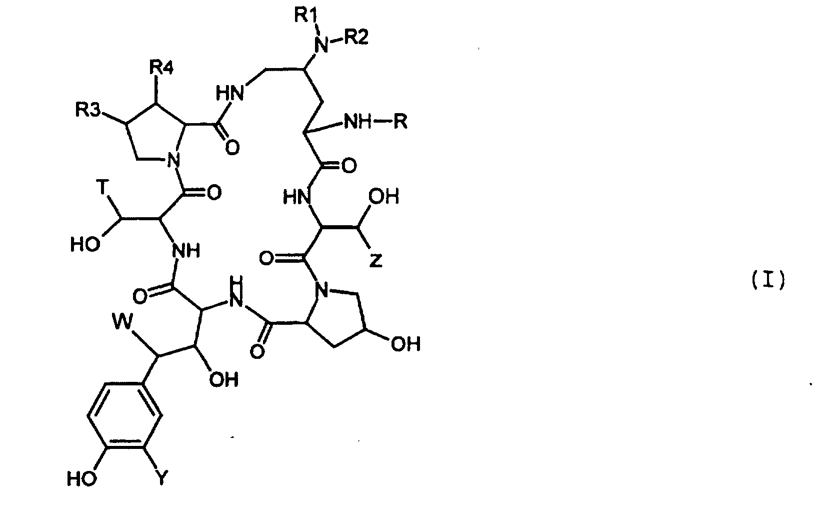 dans lesquels
ou bien R₁ et R₂ identiques ou différents l'un de l'autre, représentent un atome d'hydrogène, un radical hydroxyle, un radical alkyle renfermant jusqu'à 8 atomes de carbone linéaire, ramifié ou cyclique, éventuellement interrompu par un atome d'oxygène éventuellement substitué par un atome d'halogène, un radical OH, un radical 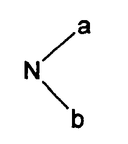 a et b identiques ou différents l'un de l'autre, représentant un atome d'hydrogène ou un radical alkyle renfermant jusqu'à 8 atomes de carbone, a et b pouvant éventuellement former avec l'atome d'azote un hétérocycle renfermant éventuellement un ou plusieurs hétéroatomes supplémentaires,
ou bien R₁ forme avec l'atome de carbone endocyclique portant le radical 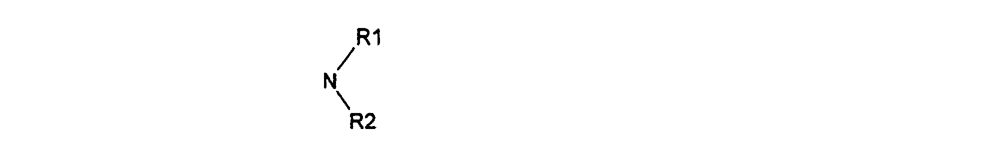 une double liaison et ou bien R2 représente un radical XRa, X représentant un atome d'oxygène ou un radical NH ou N-alkyle renfermant jusqu'à 8 atomes de carbone et Ra représente un atome d'hydrogène, un radical alkyle linéaire, ramifié ou cyclique renfermant jusqu'à 8 atomes de carbone éventuellement substitué par un ou plusieurs atomes d'halogène, par un ou plusieurs radicaux OH, CO₂H, CO₂alc, par un radical 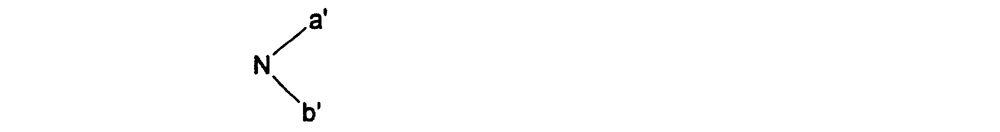 a' et b' représentant un atome d'hydrogène, un radical alkyle renfermant jusqu'à 8 atomes de carbone, a' et b' pouvant former un hétérocycle renfermant éventuellement un ou plusieurs hétéroatomes supplémentaires et/ou par un hétérocycle renfermant un ou plusieurs hétéroatomes ou R2 représente un radical 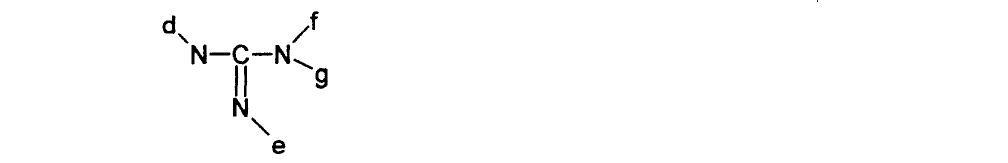 dans lequel d, e, f et g représentent un atome d'hydrogène ou un radical alkyle renfermant jusqu'à 8 atomes de carbone, f et g pouvant en outre représenter un radical acyle renfermant jusqu'à 8 atomes de carbone, e et f pouvant également former un cycle renfermant éventuellement un ou plusieurs hétéroatomes,
R₃ représente un atome d'hydrogène, un radical méthyle ou hydroxyle
R₄ représente un atome d'hydrogène ou un radical hydroxyle
R représente un radical choisi parmi les radicaux suivants : 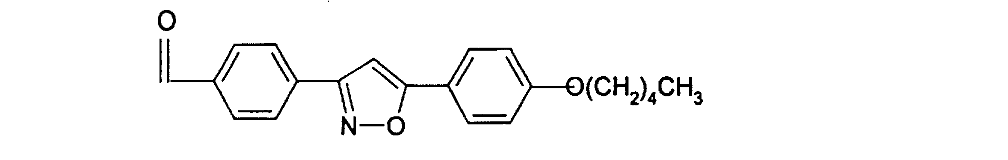 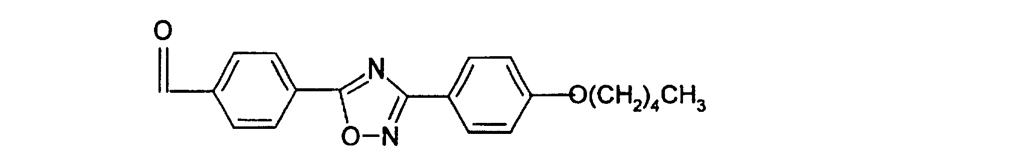 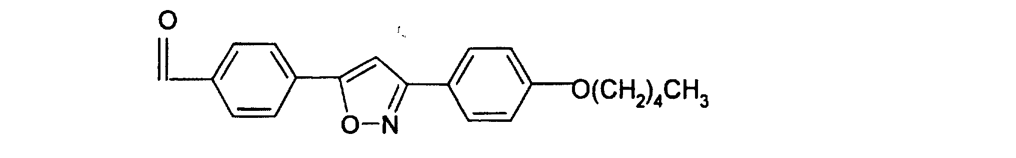 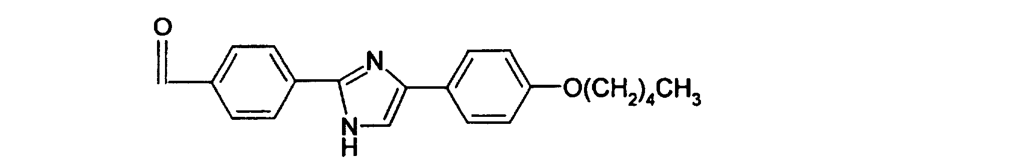 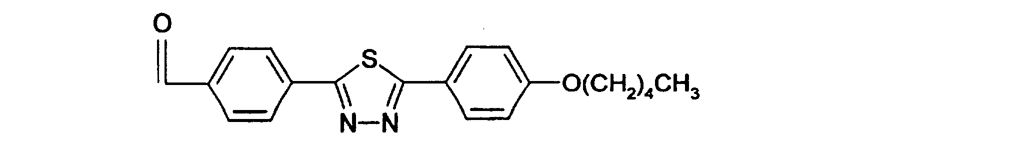 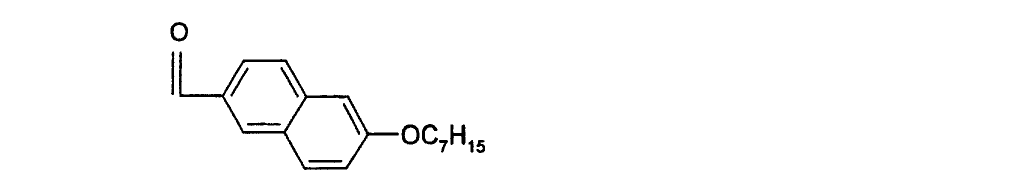 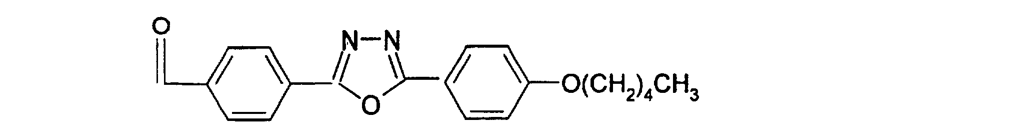 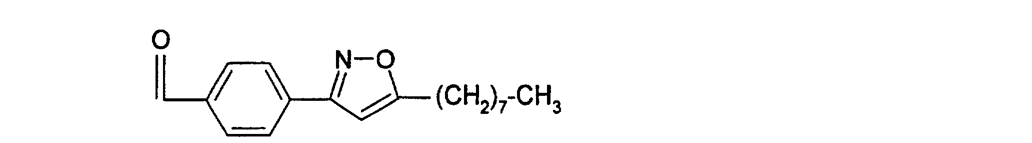 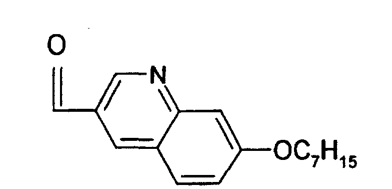 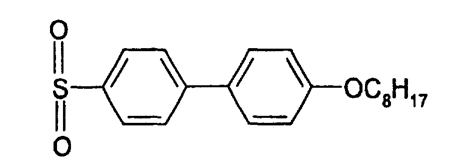 T représente un atome d'hydrogène, un radical méthyle, un radical CH₂CONH₂, CH₂C≡N, un radical (CH₂)₂NH₂ ou (CH₂)₂Nalc⁺X⁻, X étant un atome d'halogène et alc un radical alkyle renfermant jusqu'à 8 atomes de carbone,
Y représente un atome d'hydrogène, un radical hydroxyle ou un atome d'halogène ou un radical OSO₃H ou l'un des sels de ce radical,
W représente un atome d'hydrogène ou un radical OH,
Z représente un atome d'hydrogène ou un radical méthyle, ainsi que les sels d'addition avec les acides des produits de formule (I).

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane ou éthane sulfoniques, arylsulfoniques tels que les acides benzène ou paratoluènesulfoniques.

L'invention a plus particulièrement pour objet les composés de formule I dans lesquels T représente un atome d'hydrogène, ceux dans lesquels W représente un atome d'hydrogène, ceux dans lesquels Z représente un radical méthyle, ceux dans lesquels Y représente un atome d'hydrogène, ceux dans lesquels R₃ représente un radical méthyle, ceux dans lesquels R4 représente un radical hydroxyle et ceux dans lesquels R représente un radical 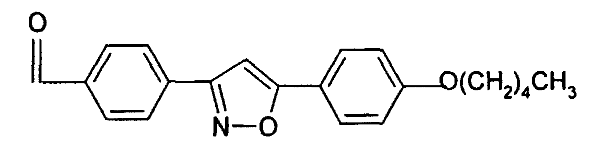 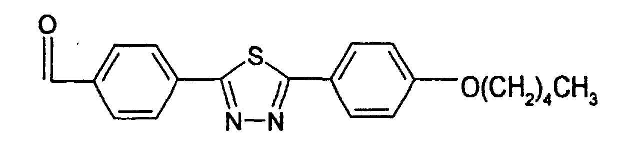 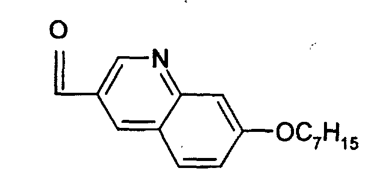 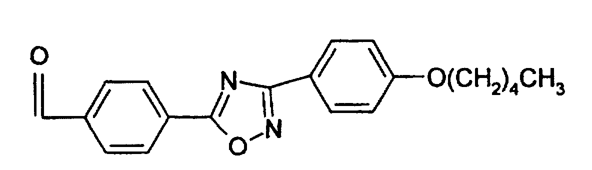 ou un radical 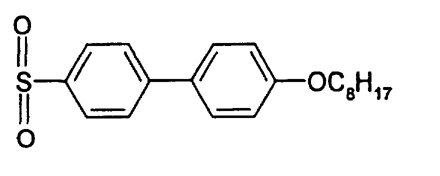 ceux dans lesquels R₁ représente un atome d'hydrogène, ceux dans lesquels R₂ représente un radical

(CH₂)₂ NH₂

ceux dans lesquels R₂ représente un radical 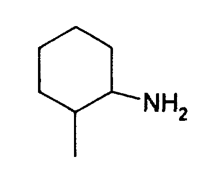 et notamment les radicaux 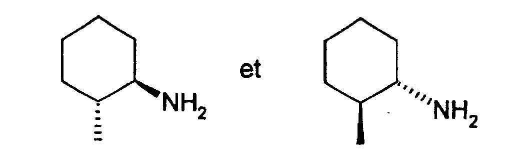 ainsi que ceux dans lesquels R2 preprésente un radical

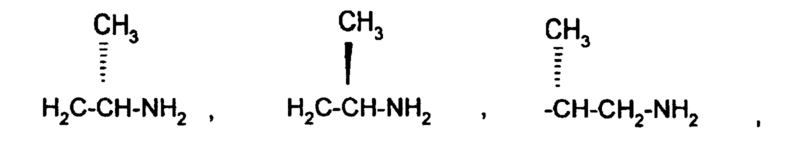

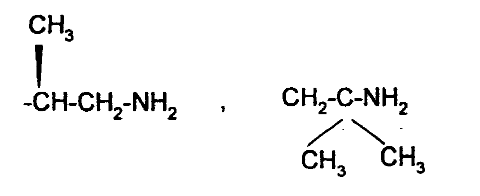

L'invention a plus particulièrement pour objet les composés de formule I dont la préparation est donnée ci-après dans la partie expérimentale.

Les composés de formule (I) présentent d'intéressantes propriétés antifongiques ; ils sont notamment actifs sur Candida albicans et autres Candida comme Candida glabrata, krusei, tropicalis, pseudotropicalis, parapsilosis et Aspergillus fumigatus, Aspergillus flavus, Cryptococcus neoformans.

Les composés de formule (I) peuvent être utilisés en tant que médicaments chez l'homme ou l'animal, pour lutter notamment contre les candidoses invasives, digestives, urinaires, vaginales ou cutanées, les cryptococcoses, par exemple les cryptococcoses neuroméningées, pulmonaires ou cutanées, les aspergilloses bronchopulmonaires et pulmonaires et les aspergilloses invasives de l'immunodéprimé.

Les composés de l'invention peuvent être utilisés également dans la prévention des affections mycosiques chez les déprimés immunitaires congénitaux ou acquis.

Les composés de l'invention ne sont pas limités à une utilisation pharmaceutique, ils peuvent être également utilisés comme fongicides dans d'autres domaines que pharmaceutiques.

L'invention a donc pour objet à titre de composés antifongiques, les composés de formule (I) ainsi que leurs sels d'addition avec les acides.

L'invention a également pour objet les composés de formule (I), à titre de médicaments.

L'invention a tout particulièrement pour objet les compositions pharmaceutiques renfermant comme principe actif au moins un composé de formule (I) ou l'un de ses sels d'addition avec les acides pharmaceutiquement acceptables.

Ces compositions peuvent être administrées par voie orale, rectale, parentérale ou par voie locale en application topique sur la peau et les muqueuses, mais la voie préférée est la voie orale ou parentérale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I) caractérisé en ce que l'on soumet un composé de formule (II) 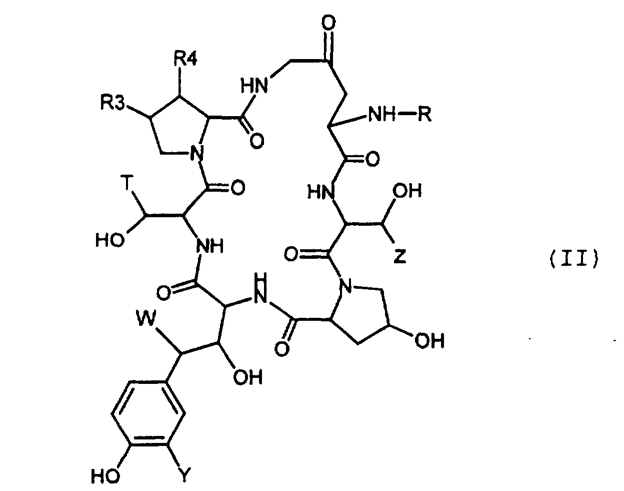 dans laquelle R, R₃, R₄, T, Y, W et Z conservent leur signification précédente, à l'action d'une amine ou d'un dérivé d'amine susceptible d'introduire le radical 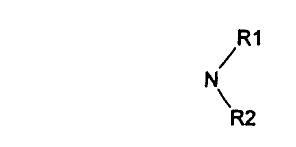 dans lequel R₁ et R₂ conservent leur signification précédente et si désiré à l'action d'un agent de réduction et/ou d'un agent de fonctionnalisation de l'amine, et/ou d'un acide pour former le sel du produit obtenu, et/ou d'un agent de séparation des différents isomères obtenus,et obtient ainsi le composé de formule (I) tel que défini ci-dessus.

Les composés de formule II mis en oeuvre sont des produits nouveaux et sont en eux-mêmes un objet de l'invention.

L'invention a également pour objet un procédé caractérisé en ce que l'on soumet un composé de formule (III) 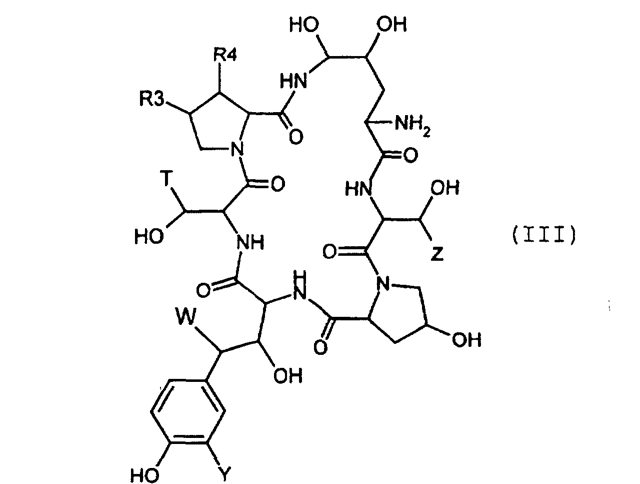 dans laquelle les différents substituants conservent leur signification précédente à l'action d'un agent capable de remplacer NH₂ par NHR, R conservant sa signification précédente pour obtenir le composé de formule (IV) 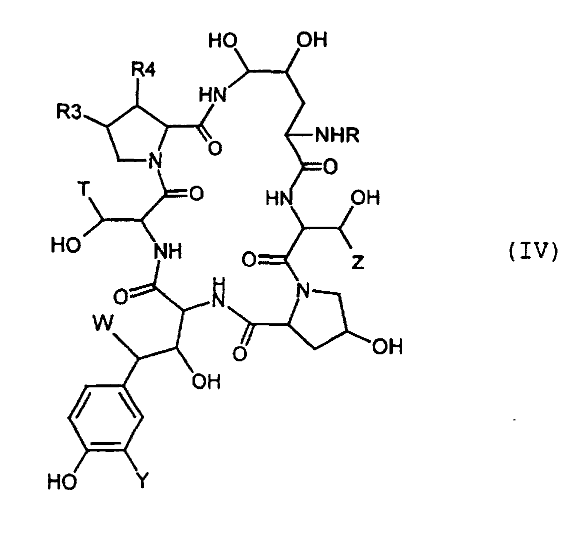 que l'on soumet à l'action de l'iodure de triméthylsilyle pour obtenir le composé de formule (II) correspondant

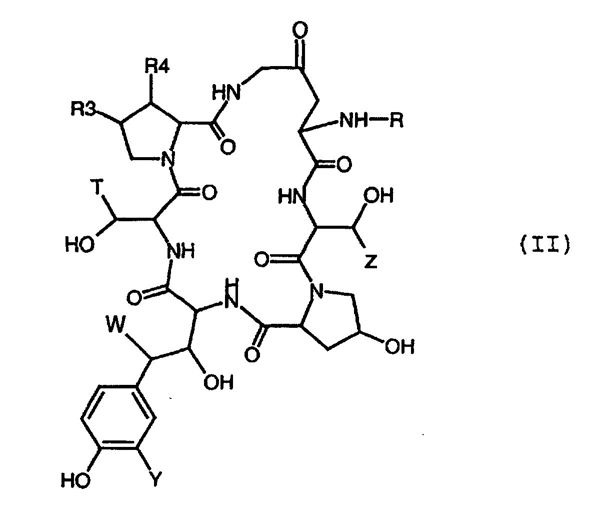

Parmi les produits de formule III et IV préférés, on peut citer tout particulièrement les produits dont la préparation est donnée ci-après dans la partie expérimentale.

Les exemples suivants illustrent l'invention sans toutefois la limiter :

### Préparation 1 : "nucléus" de déoxymulundocandine

On dissout 2 g de déoxymulundocandine dans 20 ml de DMSO. On verse cette solution dans une suspension renfermant 120 g d'Actinoplanes utahensis FH2264 dans 870 ml d'un tampon KH2PO4, K2HPO4 (pH : 6.8). On maintient le mélange réactionnel sous agitation pendant 70 heures à 30°C. On filtre. On lave le mycelium avec le tampon de phosphate (pH : 6.8). On réunit les liquides de lavage et le filtrat. On chromatographie le produit obtenu sur une résine DIAION HP 20.et obtient un produit que l'on utilise tel quel ci-après.

### EXEMPLE 1 : 1-[4-[(2-aminoéthyl)amino]-N2-[[4-[5-[4-pentyl-oxy)-phényl]-3-isoxazolyl]-phényl]-carbonyl]-L-ornithine]-4-(4-hydroxyphényl)-L-threonine] -5-L-serine-echinocandine B (isomère A et isomère B).

### STADE A : 1-[(4R,5R)-4,5-dihydroxy-N2-[[4-[5-[4-(pentyloxy)-phényl]-isoxazol-3-yl]-phényl]-carbonyl]-L-ornithine]-4-[4-(4-hydroxyphényl)-L-threonine]-5-L-serine-echinocandine B.

On introduit sous agitation et atmosphère d'azote 16,8 g du produit de la préparation 1 dans 552 ml de DMF. On agite pendant 5 minutes et ajoute 19 g d'ester de formule

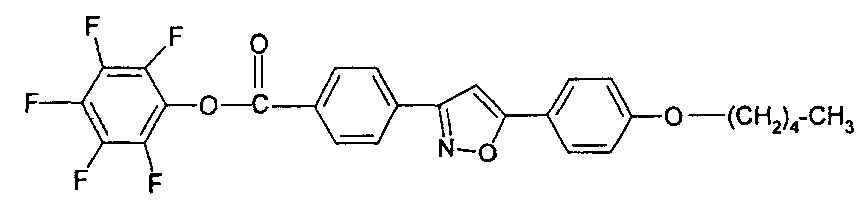

On agite pendant 29 heures. On filtre, concentre sous pression réduite. On reprend à l'éther, triture, essore, lave à l'éther éthylique, chromatographie sur silice en éluant avec le mélange chlorure de méthylène méthanol (85/15). On obtient ainsi le produit attendu rf = 0,24

### STADE B : 1-[4-oxo-N2-[[4-[5-[4-(pentyloxy)-phényl]-isoxazol-3-yl]-phényl]-carbonyl]-L-ornithine]-4-[4-(4-hydroxyphényl)-L-threonine]-5-L-serine-echinocandine B.

On ajoute 6,12 ml d'iodure de triméthylsilyle dans une suspension renfermant 16,1 g du produit du stade A et 374 ml d'acétonitrile. On chauffe ensuite 15 mn à 60°C puis on hydrolyse avec une solution saturée de thiosulfate de sodium. On amène à sec sous pression réduite puis on chromatographie sur silice en éluant avec le mélange chlorure de méthylène, méthanol, eau 86-13-1. On obtient le produit recherché rf = 0,23.
Spectre de masse
MH+ = 1083,6
Mna+ = 1105,6

### STADE C : Trifluoroacétate de 1-[4-[(2-aminoéthyl)-amino]-N2-[[4-[5-[4-(pentyloxy)-phényl]-3-isoxazol-yl]-phényl]-carbonyl]-L-ornithine]-4-[4-(4-hydroxyphényl)-L-threonine]-5-L-serine-echinocandine B (Isomère A et isomère B).

On introduit 8,6 mg de NaBH₃CN dans un mélange de 120 mg de produit du stade précédent, 2,4 ml de méthanol, 60 mg d'éthylènediamine diacétate en présence de siliporite activé 4A. On maintient le mélange réactionnel sous agitation et atmosphère d'azote pendant 18 heures. On filtre, concentre et purifie le produit obtenu par HPLC semi préparative en éluant avec le mélange acétonitrile /H₂O/TFA (40-60-0,02 %). On récupère 14,5 mg du produit recherché.
Spectre de masse
1127+ = MH+
1149+ = Mna+
On récupère : Isomère A : 14,5mg
Isomère B : 17,5 mg

### EXEMPLE 2 : Trifluoroacetate de trans-1-[4-[(2-aminocyclo-hexyl)-amino]-N2-[[4-[5-[4-(pentyloxy)-phényl]-3-isoxazolyl]-phényl]-carbonyl]-L-ornithine]-4-[4-(4-hydroxyphényl)-L-threonine]-5-L-serine-echinocandine B (Isomère A et Isomère B).

On ajoute sous agitation et sous atmosphère d'azote, jusqu'à obtention d'un pH voisin de 6, 40 µl environ d'acide acétique dans une solution renfermant 100 mg du produit obtenu au stade B de l'exemple précédent 3 ml de méthanol, 32 mg de (1R, 2R)(-)-1,2-diaminocyclohexane en présence de siliporite activé 3A. On agite pendant 5 minutes et introduit 12 mg de NaBH₃CN. On maintient le mélange réactionnel sous agitation pendant 18 heures. On filtre et concentre sous pression réduite. On purifie le produit obtenu par HPLC semi-préparative (éluant CH₃CN,H₂O,TFA 50-50-0,02 %).
Isomère A pds = 11 mg
Isomère B pds = 14 mg
Spectre de masse
1181,5 MH+

### EXEMPLE 3 : Trifluoroacetate de trans-1-[4-[(2-aminocyclo-hexyl)-amino]-N2-[[4-[5-[4-(pentyloxy)-phényl]-3-isoxazolyl]-phényl]-carbonyl]-L-ornithine]-4-[4-(4-hydroxyphényl)-L-threonine]-5-L-serine-echinocandine B (Isomère A et isomère B).

En opérant comme à l'exemple 2 avec (1S, 2S)-(-)-1,2-diaminocyclohexane, on obtient
Isomère A = 7,4 mg
Isomère B = 10,8 mg
Spectre de masse
1181,5 = MH+

### EXEMPLE 4 : Trifluoroacetate de 1-[4-[(2(S)-aminopropyl)-amine]-N2-[[4-[5-[4-(pentyloxy)-phényl]-3-isoxazolyl]-phényl]-carbonyl]-L-ornithine]-4-[4-(4-hydroxyphényl)-L-threonine]-5-L-serine-echinocandine B (Isomère A et isomère B).

En opérant comme à l'exemple 1 on a obtenu :
Isomère A : 13 mg
Isomère B : 10 mg

### EXEMPLE 5 : Trifluoroacetate de trans-1-[4-[(2-aminacyclo-hexyl)-amino]-N2-[[4-[3-[4-(pentyloxy)-phényl]-1,2,4-oxadiazol-5-yl]-phényl]-carbonyl]-L-ornithine]-4-[4-(4-hydroxyphényl)-L-threonine]-5-L-serine-echinocandine B

### STADE A : 1-[(4R,5R)-4,5-dihydroxyN2-[[4-[3-[4-(pentyloxy)-phényl]- 1,2,4-oxadiazol-5-yl]-phényl]-carbonyl]-L-ornithine]-4-[4-(4-hydroxyphényl)-L-threonine]-5-L-serine-echinocandine B.

En opérant comme à l'exemple 1 stade A, on a obtenu le produit recherché
Spectre de masse 1124 = MNa+

### STADE B : 1-[4-oxo-N2-[[4-[3-[4-(pentyloxy)-phényl]-1,2,4-oxadiazol-5-yl]-phényl]-carbonyl]-L-ornithine]-4-[4-(4-hydroxyphényl)-L-threonine]-5-L-serine-echinocandine B.

En opérant comme à l'exemple 1 stade B, on a obtenu le produit recherché.
Spectre de masse 1106,6 = MNa+
1090,8 = MH+

### STADE C : Trifluoroacetate de trans-1-[4-[(2-aminocyclo-hexyl)-amino]-N2-[[4-[3-[4-(pentyloxy)-phényl]-1,2,4-oxadiazol-5-yl]-phényl]-carbonyl]-L-ornithine]-4-[4-(4-hydroxyphényl)-L-threonine]-5-L-serine-echinocandine B.

En opérant comme à l'exemple 1 stade C, à partir de 150 mg du produit du stade B, et de 51,4 mg de (1S,2S)1,2-diaminocyclohexane, on obtient 165 mg de produit brut que l'on purifie par HPLC semi préparative (colonne KROMASIL C18) (éluant : CH₃CN-H₂O-TFA 45-55-0,1).
On obtient : Isomère A 10,8 mg
Isomère B 5,2 mg
Spectre de masse : 1204 = MNa⁺
1182 = MHa⁺

### EXEMPLE 6 : Trifluoroacétate de 1-[4-[(2-aminoéthyl)amino]-N2-[[4-[5-[4-(pentyloxy)-phényl]-1,3,4-thiadiazol-2-yl]-phényl]-carbonyl]-L-ornithine]-4-[4-(4-hydroxyphényl)-L-threonine]-5-L-serine-echinocandine B.

### STADE A : 1-[(4R,5R)-4,5-dihydroxy-N2-[(4-[3-[4-(pentyloxy)-phényl]-1,3,4-thiadiazol-2-yl]-phényl]-carbonyl]-L-ornithine]-4-[4-(4-hydroxyphényl)-L-threonine]-5-L-serine-echinocandine B.

On agite pendant 5 minutes à 20°C une suspension renfermant 2 g d'acide 4-[5-[4-(pentyloxy)-phényl]-1,3,4-thiadiazol-2-yl]-benzoique 30 ml de DMF et 30 ml de dioxanne et ajoute à O/±5°C 1,55 ml de tributylamine, 7,74 ml de chloroformiate d'isobutyle. On agite pendant 3 mn à 0±5°C puis 3 heures à la température ambiante. On introduit 4,53 g de nucleus de deoxymulundocandine obtenu comme à la préparation 1. On agite pendant 16 heures à 20°C. On concentre à sec. On reprend dans l'éther éthylique. On essore et lave à l'éther éthylique. On sèche. On obtient 7,8 g de produit que l'on purifie par chromatographie sur silice en éluant avec le mélange chlorure de méthylène-méthanol-eau 86-13-1. On obtient 2,51 g de produit recherché.

### STADE B : 1-[4-oxo-N2-[[4-[5-[4-(pentyloxy)-phényl]-1,2,4-thiadiazol-2-yl]-phényl]-carbonyl]-L-ornithine]-4-[4-(4-hydroxyphényl)-L-threonine]-5-L-serine-echinocandine B.

En opérant comme au stade B de l'exemple 1, on obtient le produit recherché.

### STADE C : 1-[4-[(2-aminoethyl)amino]-N2-[[4-[3-[4-(pentyl-oxy)-phényl]- 1,3,4-thiadiazolol-2-yl]-phényl]-carbonyl]-L-ornithine]-4-[4-(4-hydroxyphényl)-L-threonine]-5-L-serine-echinocandine B.

En opérant comme à l'exemple 1, stade C à partir du produit du stade précédent et de diacétate d'éthylènediamine, on obtient le produit recherché.
Isomère A pds = 8 mg
Isomère B pds = 9 mg

### EXEMPLE 7 : Trifluoroacétate de trans 1-[4-[(2-aminocyclo hexyl)-amino]-N2-[[4-[5-[4-(pentyloxy)-phényl]-1,3,4-thiadiazol-2-yl]-phényl]-carbonyl]-L-ornithine]-4-[4-(4-hydroxyphényl)-L-threonine]-5-L-serine-echinocandine B

En opérant comme à l'exemple 1, à partir du produit du stade B de l'exemple 5 (50 mg) et de (1S,2S)(+)1,2-diaminocyclohexane (15,6 mg), on obtient le produit recherché.
Isomère A = 4 mg
Isomère B = 6,5 mg

### EXEMPLE 8 : Trifluoroacétate de trans 1-[4-[(2-aminocyclo-hexyl)-amino]-N2-[[4-[5-[4-(pentyloxy)-phényl]-1,2,4-thiadiazol-2-yl]-phényl]-carbonyl]-L-ornithine]-4-[4-(4-hydroxyphényl)-L-threonine]-5-L-serine-echinocandine B (Isomère A et isomère B)

En opérant comme à l'exemple 1 stade C à partir du produit du stade B de l'exemple 5 (50 mg) et du (1R,2R)-1,2-diaminocyclohexane (15,6 mg), on obtient le produit recherché.
Isomère A = 8,8 mg
Isomère B = 10,6 mg

### EXEMPLE : Composition pharmaceutique :

On a préparé des comprimés renfermant :
- Produit de l'exemple 1 150 mg
- Excipient q.s.p. 1 g
(Détail de l'excipient : amidon, talc, stéarate de magnésium).

### ETUDE PHARMRCOLOGIQUE

### A - Inhibition de la glucane synthase de Candida albicans.

On purifie des membranes de Candida albicans selon le procédé décrit par Tang et al. Antimicrob. Agents Chemother, 35, 99-103, 1991. 22,5 µg de protéines membranaires sont incubées dans un mélange de 2Mm de 14C-UDP glucose (activité spécifique = 0,34 mCi./mmol, 50 µg d'α-amylase, 1Mm de dithiotreitol (DTT), 1Mm EDTA, 100Mm NaF, 7µM de GTP-γ-S, 1M de sucrose et 50Mm DE Tris-HCL (pH 7,8) dans un volume de 100µl. Le milieu est incubé à 25°C pendant 1 heure et la réaction terminée par addition de TCA à une concentration finale de 5%. Le mélange réactionnel est transféré sur un filtre de fibre de verre pré-humidifié. Le filtre est lavé, séché et sa radioactivité est comptée.

La mulundocandine est utilisé comme contrôle positif.

Le contrôle du véhicule est effectué avec la même quantité de DMSO 1%. Les résultats obtenus montrent que les produits de l'invention présentent sur ce test une bonne activité en particulier les produits de l'exemple 1.

### B - activité sur l'enzyme d'Aspergillus fumigatus.

L'enzyme est préparée selon le procédé de Beaulieu et al. (Antimicrob. Agents Chemother. 38, 937-944, 1994).

Le protocole utilisé est identique au protocole décrit ci-dessus pour l'enzyme de Candida albicans sauf que l'on n'utilise pas de dithiotreitol dans le mélange réactionnel.

Les produits présentent sur ce test une bonne activité.

## Revendications

1. Sous toutes les formes d'isomères possibles ainsi que leurs mélanges, les composés de formule (I) : 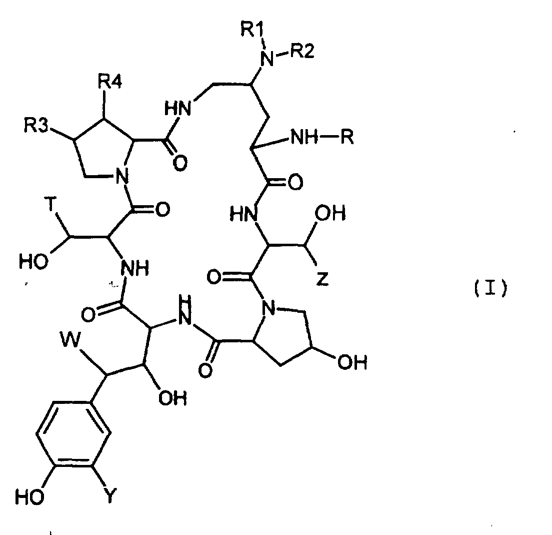 dans lesquels
ou bien R₁ et R₂ identiques ou différents l'un de l'autre, représentent un atome d'hydrogène, un radical hydroxyle, un radical alkyle renfermant jusqu'à 8 atomes de carbone linéaire, ramifié ou cyclique, éventuellement interrompu par un atome d'oxygène éventuellement substitué par un atome d'halogène, un radical OH, un radical 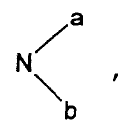 a et b identiques ou différents l'un de l'autre,
représentant un atome d'hydrogène ou un radical alkyle renfermant jusqu'à 8 atomes de carbone, a et b pouvant éventuellement former avec l'atome d'azote un hétérocycle renfermant éventuellement un ou plusieurs hétéroatomes supplémentaires,
ou bien R₁ forme avec l'atome de carbone endocyclique portant le radical 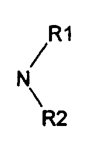 une double liaison et ou bien R2 représente un radical XRa, X représentant un atome d'oxygène
ou un radical NH ou N-alkyle renfermant jusqu'à 8 atomes de carbone et Ra représente un atome d'hydrogène, un radical alkyle linéaire, ramifié ou cyclique renfermant jusqu'à 8 atomes de carbone éventuellement substitué par un ou plusieurs atomes d'halogène, par un ou plusieurs radicaux OH, CO₂H, CO₂alc, par un radical 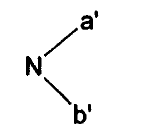 a' et b' représentant un atome d'hydrogène, un radical alkyle renfermant jusqu'à 8 atomes de carbone, a' et b' pouvant former un hétérocycle renfermant éventuellement un ou plusieurs hétéroatomes supplémentaires et/ou par un hétérocycle renfermant un ou plusieurs hétéroatomes ou R₂ représente un radical 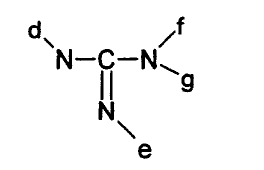 dans lequel d, e, f et g représentent un atome d'hydrogène ou un radical alkyle renfermant jusqu'à 8 atomes de carbone, f et g pouvant en outre représenter un radical acyle renfermant jusqu'à 8 atomes de carbone, e et f pouvant également former un cycle renfermant éventuellement un ou plusieurs hétéroatomes,
R₃ représente un atome d'hydrogène, un radical méthyle ou hydroxyle
R₄ représente un atome d'hydrogène ou un radical hydroxyle
R représente un radical choisi parmi les radicaux suivants : 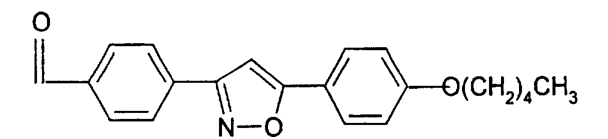 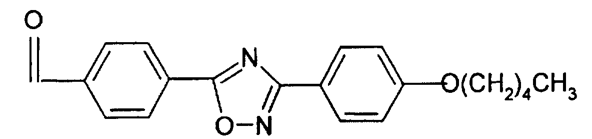 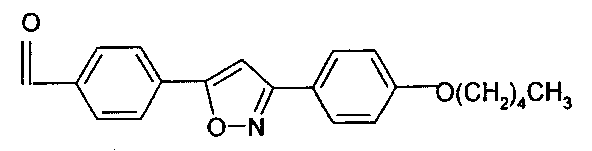 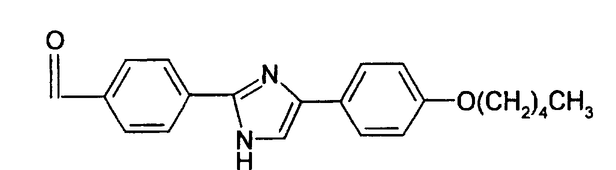 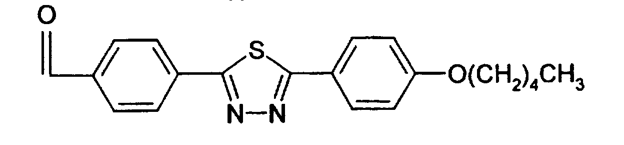 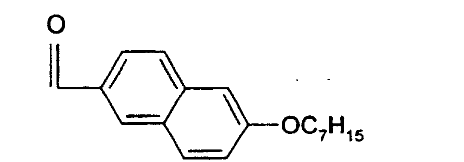 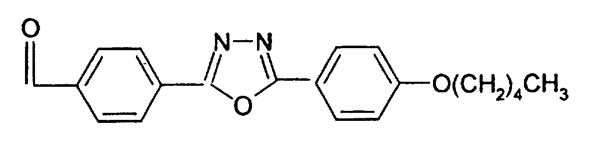 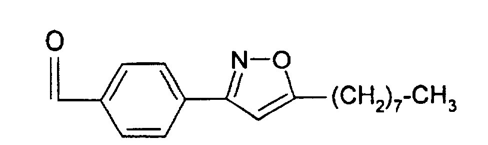 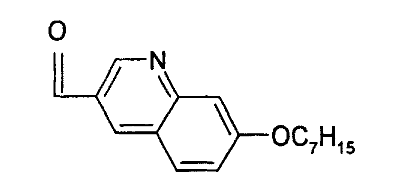 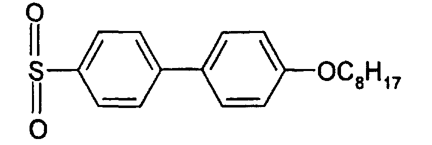 T représente un atome d'hydrogène, un radical méthyle, un radical CH₂CONH₂, CH₂CN, un radical (CH₂)₂NH₂ ou (CH₂)₂Nalc⁺X⁻, X étant un atome d'halogène et alc un radical alkyle renfermant jusqu'à 8 atomes de carbone,
Y représente un atome d'hydrogène, un radical hydroxyle ou un atome d'halogène ou un radical OSO₃H ou l'un des sels de ce radical,
W représente un atome d'hydrogène ou un radical OH,
Z représente un atome d'hydrogène ou un radical méthyle,
ainsi que les sels d'addition avec les acides des produits de formule (I).

2. Les composés de formule (I) définis à la revendication 1 dans lesquels T représente un atome d'hydrogène.

3. Les composés de formule (I) définis à la revendication 1
ou 2 dans lesquels W représente un atome d'hydrogène.

4. Les composés de formule (I) définis à l'une quelconque des revendications 1 à 3, dans lesquels Z représente un radical méthyle.

5. Les composés de formule (I) définis à l'une quelconque des revendications 1 à 4 dans lesquels Y représente un atome d'hydrogène.

6. Les composés de formule (I) définis à l'une quelconque des revendications 1 à 5 dans lesquels R₃ représente un radical méthyle.

7. Les composés de formule définis à l'une quelconque des revendications 1 à 6 dans lesquels R₄ représente un radical d'hydroxyle.

8. Les composés de formule (I) définis à l'une quelconque des revendications 1 à 7 dans lesquels R représente un radical 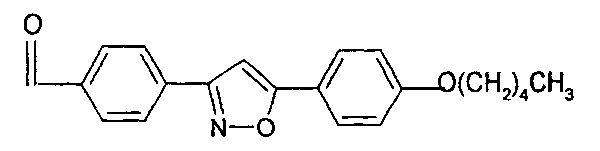 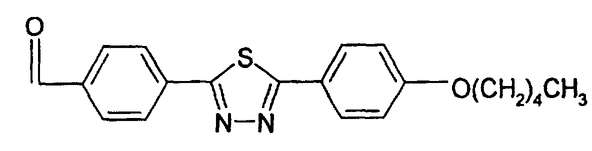 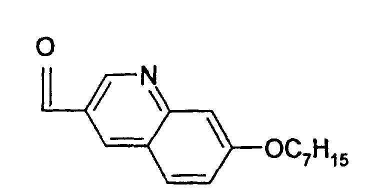 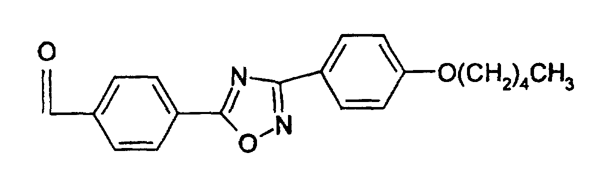 ou un radical

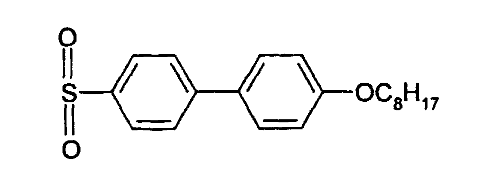

9. Les composés de formule I définis à l'une quelconque des revendications 1 à 8 dans lesquels R₁ représente un radical d'hydrogène.

10. Les composés de formule I définis à l'une quelconque des renvendications 1 à 9 dans lesquels R₂ représente un radical
(CH₂)₂ NH₂

11. Les composés de formule I définis à l'une quelconque des revendications 1 à 9 dans lesquels R₂ représente un radical 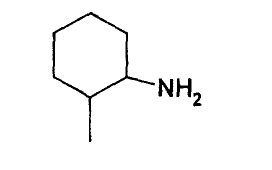 et notamment les radicaux

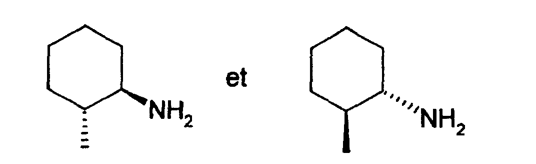

12. Les composés de formule I définis à l'une quelconque des revendications 1 à 9 dans lesquels R₂ représente un radical

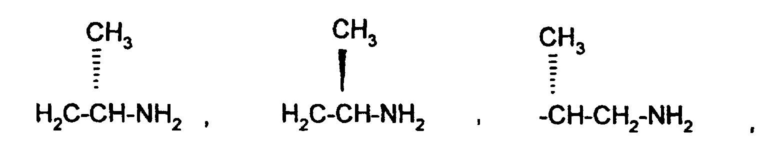

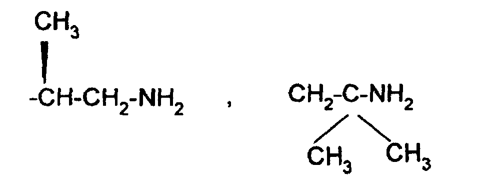

13. Les composés de formule I définis à la revendication 1 dont les noms suivent :
- Trifluoroacétate de 1-[4-[(2-aminoéthyl)-amino]-N2-[[4-[5-[4-(pentyloxy)-phényl]-3-isoxazolyl]-phényl]-carbonyl]-L-ornithine]-4-[4-(4-hydroxyphényl)-L-threonine]-5-L-serine-echinocandine B,
- Trifluoroacétate de trans-1-[4-[(2-aminocyclohexyl)-amino]-N2-[[4-[5-[4-(pentyloxy)-phényl]-3-isoxazolyl]-phényl]-carbonyl]-L-ornithine]-4-[4-(4-hydroxyphényl)-L-threonine]-5-L-serine-echinocandine B,
- Trifluoroacétate de 1-[4-[(2(S)-aminopropyl)-amino]-N2-[[4-[5-[4-(pentyloxy)-phényl]-3-isoxazolyl]-phényl]-carbonyl]-L-ornithine]-4-[4-(4-hydroxyphényl]-L-threonine]-5-L-serine-echinocandine B,
- Trifluoroacétate de 1-[4-[(2-aminoéthyl)amino]-N2-[[4-[5-[4-(pentyloxy)-phényl]-1,3,4-thiadiazol-2-yl]-phényl]-carbonyl]-L-ornithine]-4-[4-(4-hydroxyphényl)-L-threonine]-5-L-serine-echinocandine B,
- Trifluoroacétate de trans 1-[4-[(2-aminocyclohexyl)-amino]-N2-[[4-[5-[4-(pentyloxy)-phényl]-1,3,4-thiadiazol-2-yl]-phényl]-carbonyl]-L-ornithine]-4-[4-(4-hydroxyphényl)-L-threonine]-5-L-serine-echinocandine B,
- Trifluoroacétate de trans 1-[4-[(2-aminocyclohexyl)-amino]-N2-[[4-[3-[4-(pentyloxy)-phényl]-1,2,4-oxadiazol-5-yl]-phényl]-carbonyl]-L-ornithine]-4-[4-(4-hydroxyphényl)-L-threonine]-5-L-serine-echinocandine B.

14. Procédé de préparation des composés de formule (I) définis à l'une quelconque des revendications 1 à 13, **caractérisé en ce que** l'on soumet un composé de formule (II) 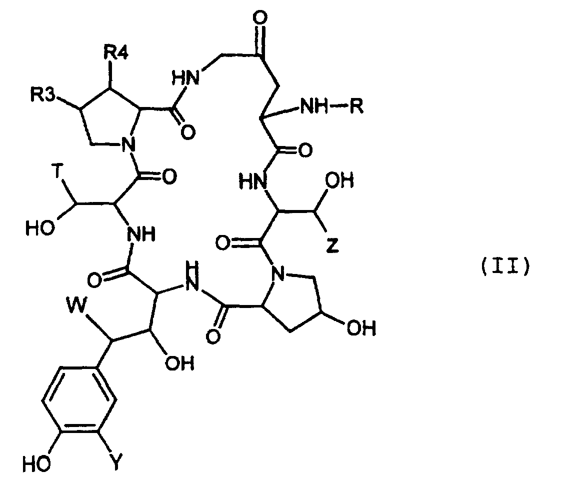 dans laquelle R, R₃, R₄, T, Y, W et Z conservent la signification donnée dans les revendication précédentes , à l'action d'une amine ou d'un dérivé d'amine susceptible d'introduire le radical 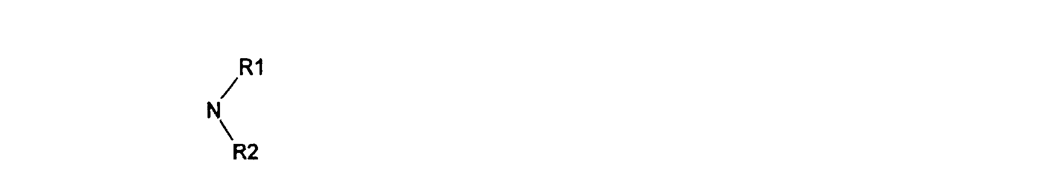 dans lequel R1 et R2 conservent la signification donnée dans les revendications précédentes et si désiré à l'action d'un agent de réduction
et/ou d'un agent de fonctionnalisation de l'amine,
et/ou d'un acide pour former le sel du produit obtenu,
et/ou d'un agent de séparation des différents isomères obtenus,
et obtient ainsi le composé de formule (I) tel que défini à la revendication 1.

15. A titre de produits chimiques nouveaux, les composés de formule (II) définis à la revendication 14.

16. Procédé selon la revendication 14 **caractérisé en ce que** l'on soumet un composé de formule (III) 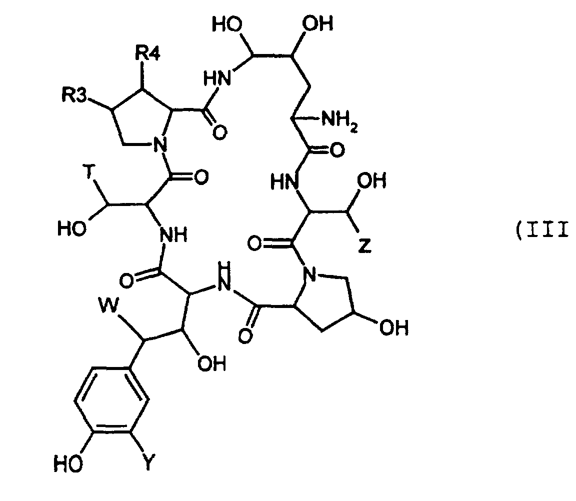 dans laquelle les différents substituants conservent la signification donnée dans les revendications précédentes à l'action d'un agent capable de remplacer NH₂ par NHR, R conservant sa signification précédente pour obtenir le composé de formule (IV) 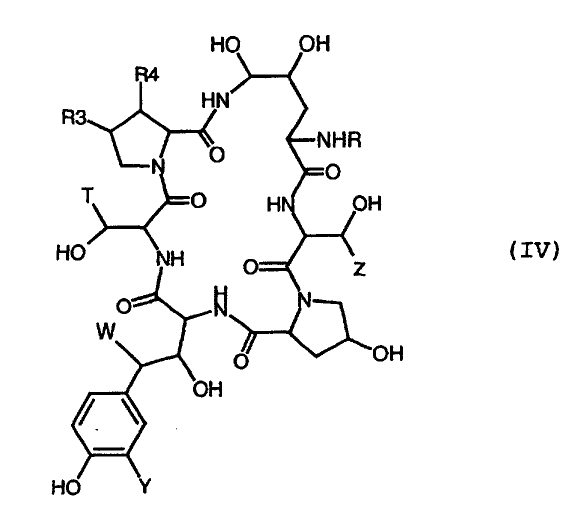 que l'on soumet à l'action de l'iodure de triméthylsilyle pour obtenir le composé de formule (II) correspondant

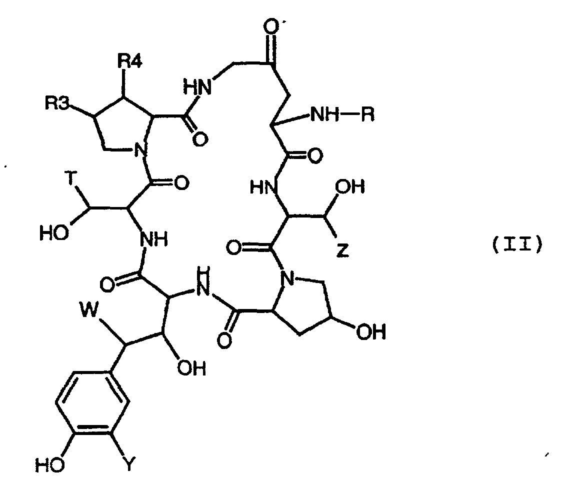

17. A titre de composés antifongiques, les composés de formule (I) définis à l'une quelconque des revendications 1 à 13, ainsi que leurs sels d'addition avec les acides.

18. Les compositions pharmaceutiques renfermant à titre de médicament au moins un composé de formule (I) défini à l'une quelconque des revendications 1 à 13, ainsi que leurs sels d'addition avec les acides pharmaceutiquement acceptables.

## Claims

1. In all possible isomeric forms as well as their mixtures, the compounds of formula (I): 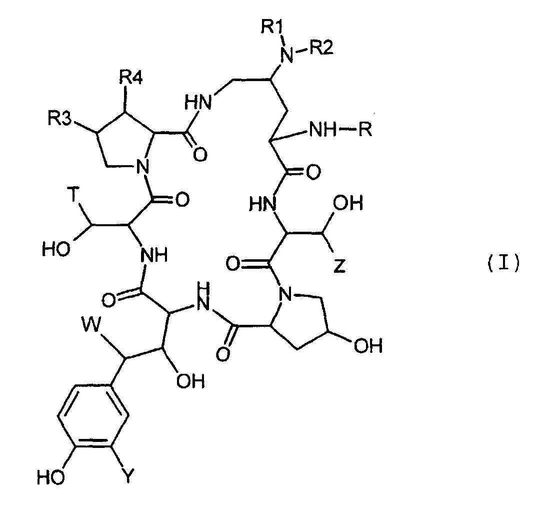 in which
either R₁ and R₂ which are identical to or different from one another, represent a hydrogen atom, a hydroxyl radical, a linear, branched or cyclic alkyl radical containing up to 8 carbon atoms optionally interrupted by an oxygen atom optionally substituted by a halogen atom, an OH radical, an 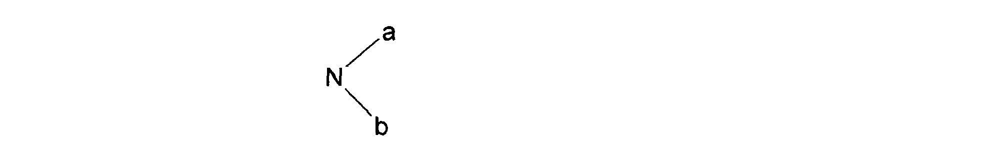 radical, a and b being
identical to or different from one another,
representing a hydrogen atom or an alkyl radical containing up to 8 carbon atoms, a and b can optionally form with the nitrogen atom a heterocycle optionally containing one or more additional heteroatoms,
or R₁ forms with the endocyclic carbon atom carrying the 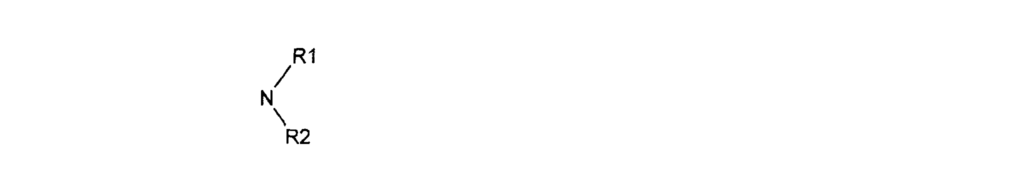 radical a double bond or R2 represents an Xra radical, X representing an oxygen atom or an NH or N-alkyl radical containing up to 8 carbon atoms and Ra represents a hydrogen atom, a linear, branched or cyclic alkyl radical containing up to 8 carbon atoms optionally substituted by one or more halogen atoms, by one or more OH, CO₂H, CO₂alk radicals, by an 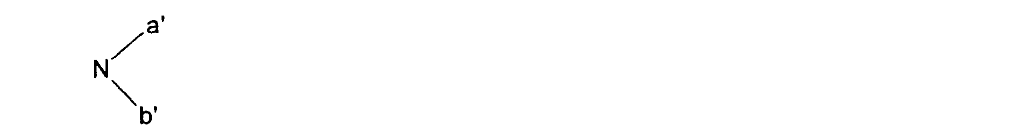 radical, a' and b' representing a hydrogen atom, an alkyl radical containing up to 8 carbon atoms, a' and b' can form a heterocycle optionally containing one or more additional heteroatoms and/or by a heterocycle containing one or more heteroatoms or R₂ represents a 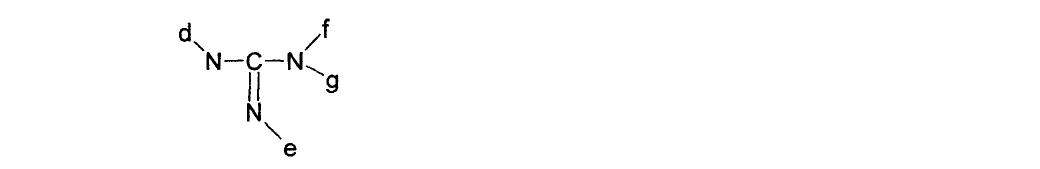 radical in which d, e, f and g represent a hydrogen atom or an alkyl radical containing up to 8 carbon atoms, f and g can moreover represent an acyl radical containing up to 8 carbon atoms, e and f can also form a ring optionally containing one or more heteroatoms,
R₃ represents a hydrogen atom, a methyl or hydroxyl radical
R₄ represents a hydrogen atom or a hydroxyl radical
R represents a radical chosen from the following radicals: 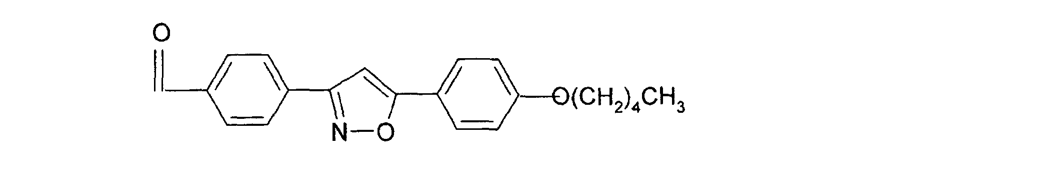 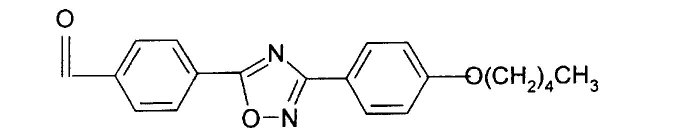 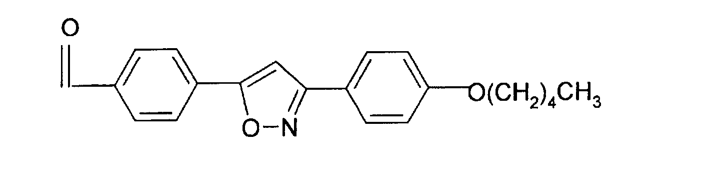 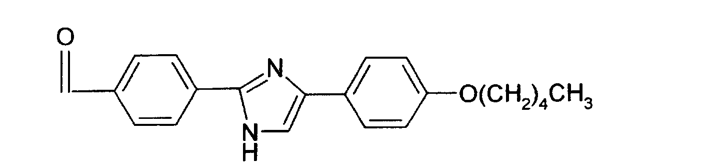 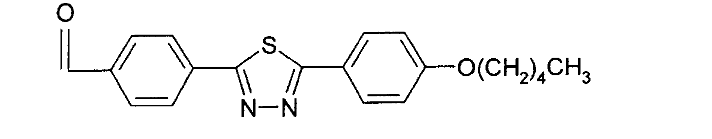 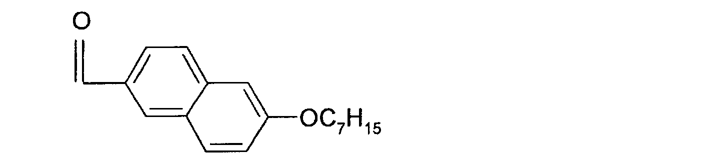 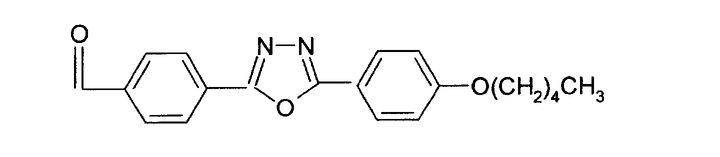 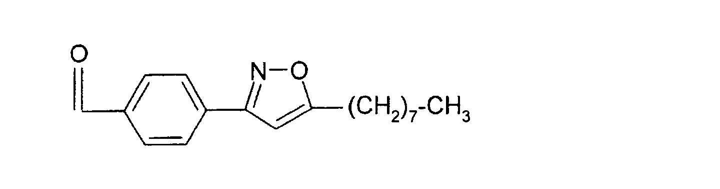 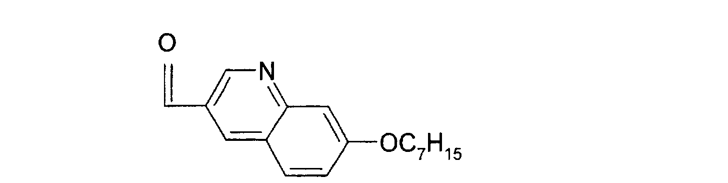 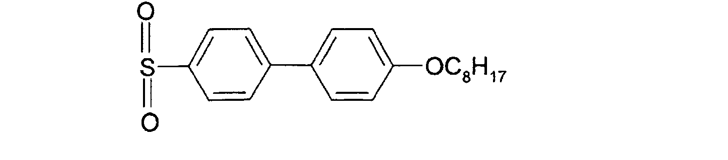 T represents a hydrogen atom, a methyl radical, a CH₂CONH₂, CH₂CN radical, a (CH₂)₂NH₂ or (CH₂)₂Nalc⁺X⁻ radical, X being a halogen atom and alk an alkyl radical containing up to 8 carbon atoms,
Y represents a hydrogen atom, a hydroxyl radical or a halogen atom or an OSO₃H radical or one of the salts of this radical,
W represents a hydrogen atom or an OH radical,
Z represents a hydrogen atom or a methyl radical,
as well as the addition salts with acids of products of formula (I).

2. The compounds of formula (I) defined in claim 1 in which T represents a hydrogen atom.

3. The compounds of formula (I) defined in claim 1 or 2 in which W represents a hydrogen atom.

4. The compounds of formula (I) defined in any one of claims 1 to 3, in which Z represents a methyl radical.

5. The compounds of formula (I) defined in any one of claims 1 to 4 in which Y represents a hydrogen atom.

6. The compounds of formula (I) defined in any one of claims 1 to 5 in which R₃ represents a methyl radical.

7. The compounds of formula defined in any one of claims 1 to 6 in which R₄ represents a hydroxyl radical.

8. The compounds of formula (I) defined in any one of claims 1 to 7 in which R represents a 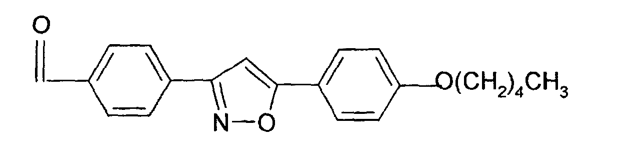 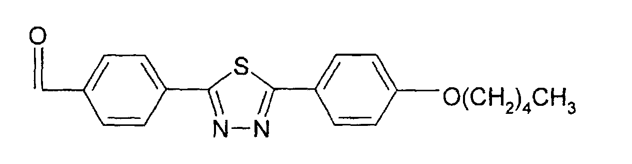 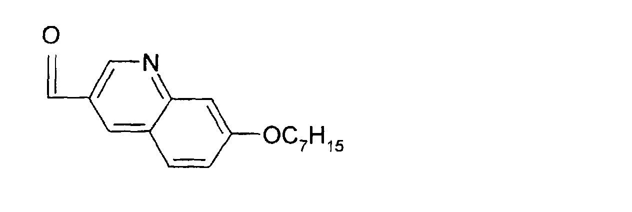 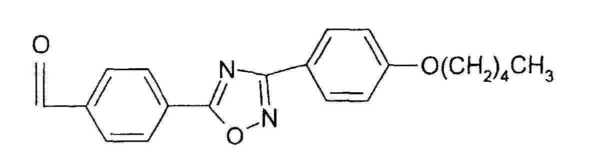 radical or a 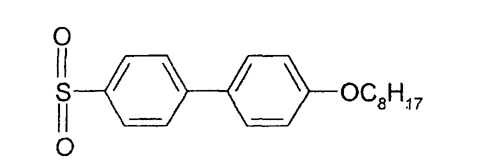 radical.

9. The compounds of formula I defined in any one of claims 1 to 8 in which R₁ represents a hydrogen radical.

10. The compounds of formula I defined in any one of claims 1 to 9 in which R₂ represents a
(CH₂)₂ NH₂
radical.

11. The compounds of formula I defined in any one of claims 1 to 9 in which R₂ represents a 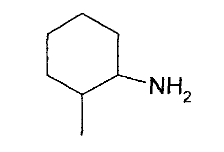 radical and in particular the 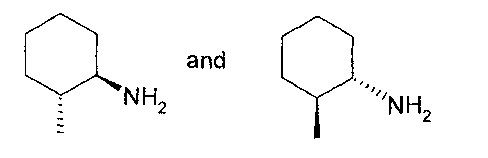 radicals.

12. The compounds of formula I defined in any one of claims 1 to 9 in which R₂ represents a 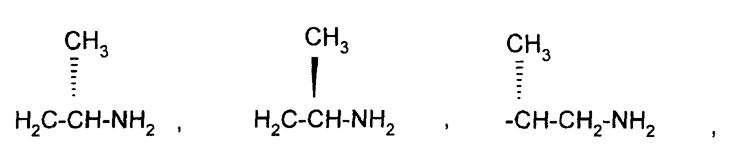 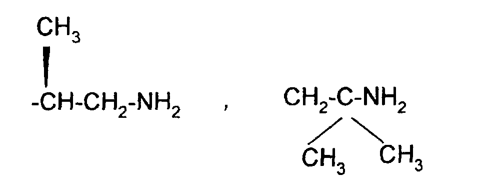 radical.

13. The compounds of formula I defined in claim 1 the names of which follow:
- 1-[4-[(2-aminoethyl)-amino]-N2-[[4-[5-[4-(pentyloxy)-phenyl]-3-isoxazolyl]-phenyl]-carbonyl]-L-ornithine]-4-[4-(4-hydroxyphenyl)-L-threonine]-5-L-serine-echinocandin B trifluoroacetate,
- trans-1-[4-[(2-aminocyclohexyl)-amino]-N2-[[4-[5-[4-(pentyloxy)-phenyl]-3-isoxazolyl]-phenyl]-carbonyl]-L-ornithine]-4-[4-(4-hydroxyphenyl)-L-threonine]-5-L-serine-echinocandin B trifluoroacetate,
- 1-[4-[(2(S)-aminopropyl)-amino]-N2-[[4-[5-[4-(pentyloxy)-phenyl]-3-isoxazolyl]-phenyl]-carbonyl]-L-ornithine]-4-[4-(4-hydroxyphenyl)-L-threonine]-5-L-serine-echinocandin B trifluoroacetate,
- 1-[4-[(2-aminoethyl)amino]-N2-[[4-[5-[4-(pentyloxy)-phenyl]-1,3,4-thiadiazol-2-yl]-phenyl]-carbonyl]-L-ornithine]-4-[4-(4-hydroxyphenyl)-L-threonine]-5-L-serine-echinocandin B trifluoroacetate,
- trans 1-[4-[(2-aminocyclohexyl)-amino]-N2-[[4-[5-[4-(pentyloxy)-phenyl]-1,3,4-thiadiazol-2-yl]-phenyl]-carbonyl]-L-ornithine]-4-[4-(4-hydroxyphenyl)-L-threonine]-5-L-serine-echinocandin B trifluoroacetate,
- trans 1-[4-[(2-aminocyclohexyl)-amino]-N2-[[4-(3-[4-(pentyloxy)-phenyl]-1,2,4-oxadiazol-5-yl]-phenyl]-carbonyl]-L-ornithine]-4-[4-(4-hydroxyphenyl)-L-threonine]-5-L-serine-echinocandin B trifluoroacetate.

14. Process for the preparation of compounds of formula (I) defined in any one of claims 1 to 13, **characterized in that** a compound of formula (II) 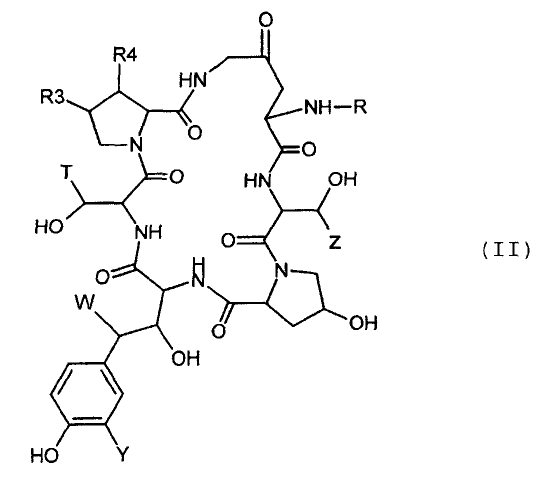 in which R, R₃, R₄, T, Y, W and Z retain the meaning given in the preceding claims, is subjected to the action of an amine or an amine derivative capable of introducing the 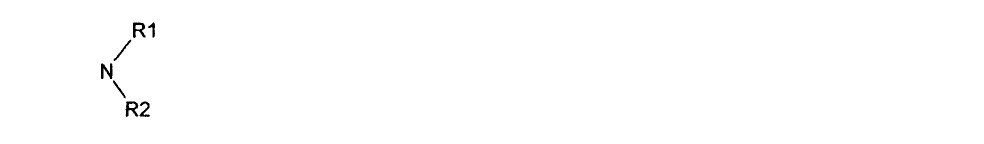 radical in which R1and R2 retain the meaning given in the preceding claims and if desired to the action of a reducing agent
and/or of an amine functionalization agent,
and/or of an acid in order to form the salt of the product obtained,
and/or of a separation agent of the different isomers obtained,
and in this way the compound of formula (I) as defined in claim 1 is obtained.

15. As new chemical products, the compounds of formula (II) defined in claim 14.

16. Process according to claim 14 **characterized in that** a compound of formula (III) 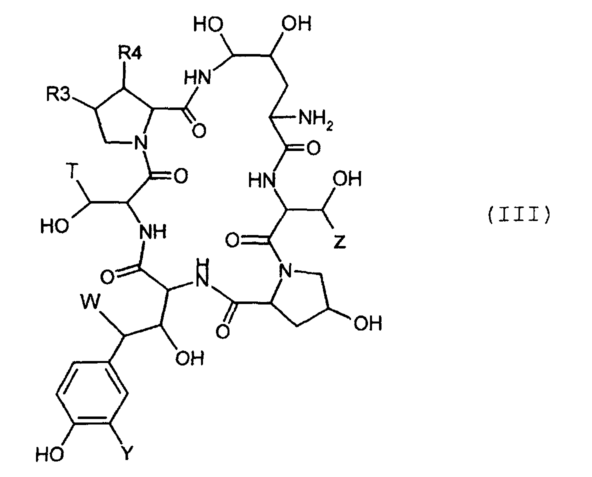 in which the different substituents retain the meaning given in the preceding claims is subjected to the action of an agent capable of replacing NH₂ by NHR, R retaining its preceding meaning in order to obtain the compound of formula (IV) 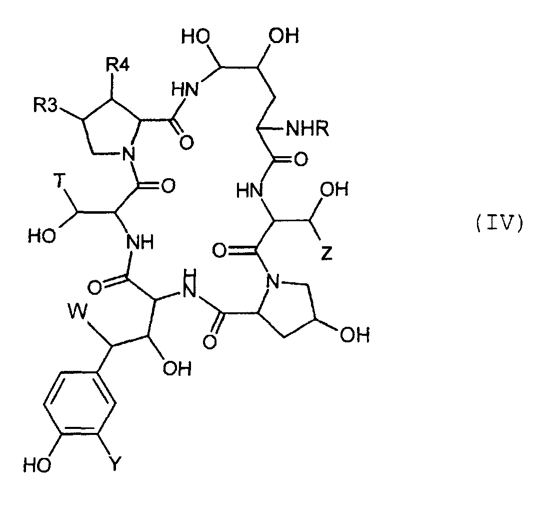 which is subjected to the action of trimethylsilyl iodine in order to obtain the corresponding compound of formula (II)

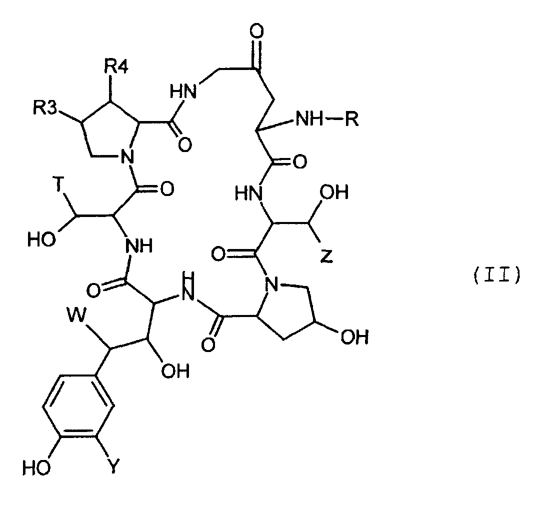

17. As antifungal compounds, the compounds of formula (I) defined in any one of claims 1 to 13, as well as their addition salts with acids.

18. The pharmaceutical compositions containing as medicament at least one compound of formula (I) defined in any one of claims 1 to 13, as well as their addition salts with pharmaceutically acceptable acids.

## Patentansprüche

1. In allen möglichen Isomerformen sowie ihren Gemischen die Verbindungen der Formel (I): 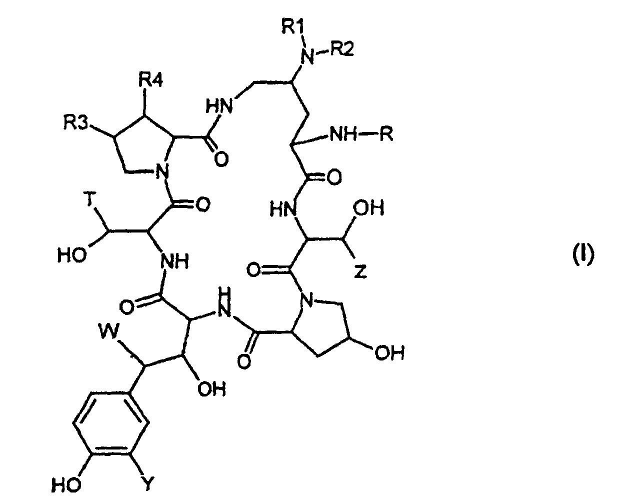 worin
entweder R₁ und R₂, die gleich oder voneinander verschieden sind, darstellen ein Wasserstoffatom, einen Hydroxylrest, einen linearen, verzweigten oder cyclischen Alkylrest mit bis zu 8 Kohlenstoffatomen, gegebenenfalls unterbrochen durch ein Sauerstoffatom, gegebenenfalls substituiert mit einem Halogenatom, einem OH-Rest, einem 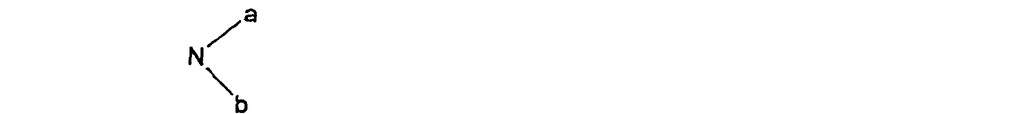 -Rest, wobei a und b, die gleich oder voneinander verschieden sind, ein Wasserstoffatom oder einen Alkylrest mit bis zu 8 Kohlenstoffatomen darstellen, wobei a und b gegebenenfalls mit dem Stickstoffatom einen Heterocyclus bilden können, der gegebenenfalls ein oder mehrere zusätzliche Heteroatome enthält, oder R₁ mit dem endocyclischen Kohlenstoffatom, das den 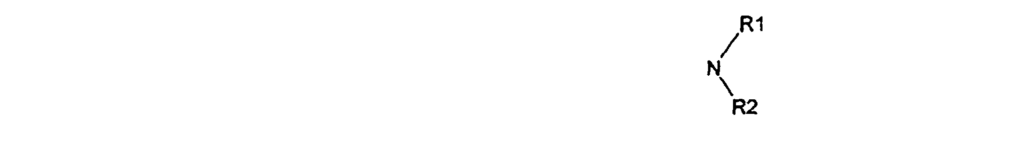 -Rest trägt, eine Doppelbindung bildet und entweder R₂ einen XRa-Rest darstellt, wobei X ein Sauerstoffatom oder einen NH- oder N-Alkylrest mit bis zu 8 Kohlenstoffatomen darstellt und Ra darstellt ein Wasserstoffatom, einen linearen, verzweigten oder cyclischen Alkylrest mit bis zu 8 Kohlenstoffatomen, gegebenenfalls substituiert mit einem oder mehreren Halogenatomen, mit einem oder mehreren OH-, CO₂H-, CO₂alc-Resten, mit einem Rest 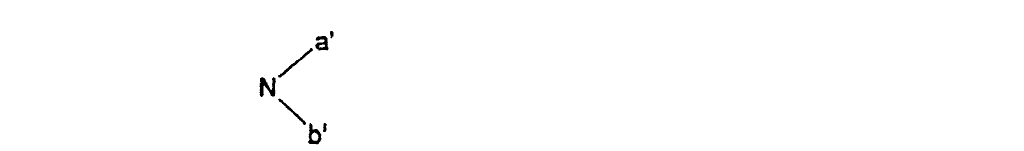 wobei a' und b' ein Wasserstoffatom, einen Alkylrest mit bis zu 8 Kohlenstoffatomen darstellen, wobei a' und b' einen Heterocyclus bilden können, der gegebenenfalls ein oder mehrere zusätzliche Heteroatome enthält, und/oder mit einem Heterocyclus, der ein oder mehrere Heteroatome enthält, oder R₂ einen Rest 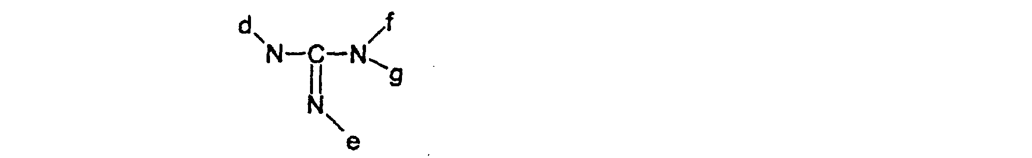 darstellt, worin d, e, f und g ein Wasserstoffatom oder einen Alkylrest mit bis zu 8 Kohlenstoffatomen darstellen, wobei f und g außerdem einen Acylrest mit bis zu 8 Kohlenstoffatomen darstellen können, wobei e und f auch einen Ring bilden können, der gegebenenfalls ein oder mehrere Heteroatome enthält,
R₃ ein Wasserstoffatom, einen Methyl- oder Hydroxylrest darstellt,
R₄ ein Wasserstoffatom oder einen Hydroxylrest darstellt,
R einen Rest darstellt, der unter den folgenden Resten ausgewählt ist: 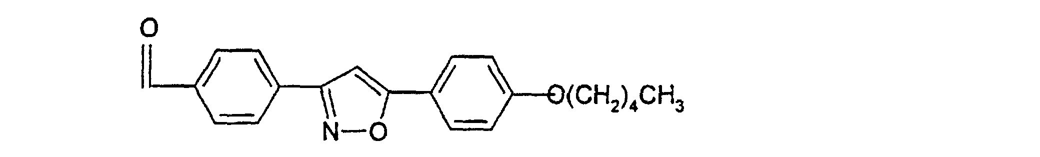 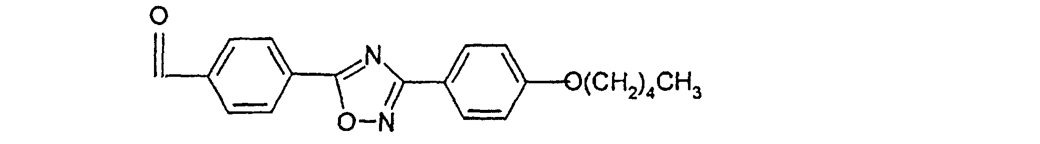 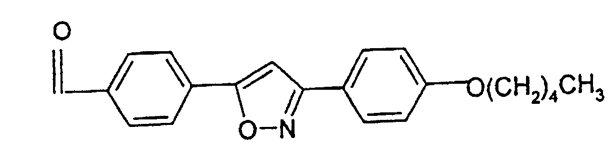 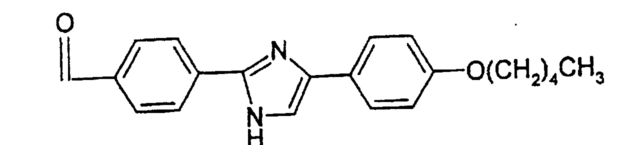 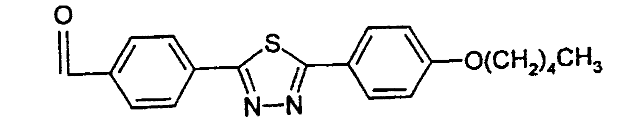 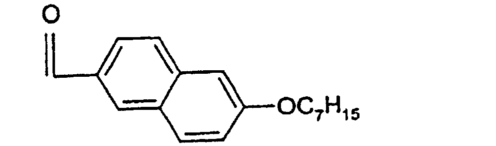 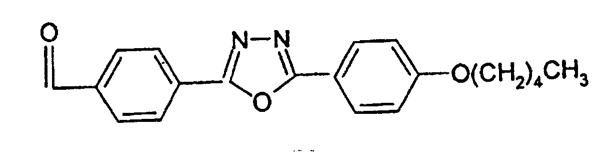 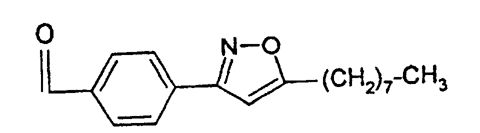 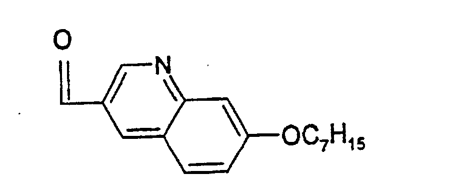 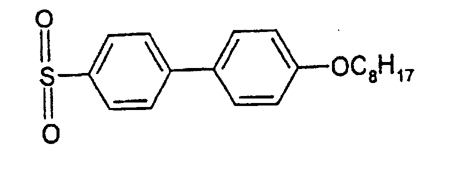 T ein Wasserstoffatom, einen Methylrest, einen CH₂CONH₂-, CH₂CN-Rest, einen (CH₂)₂NH₂- oder (CH₂)₂Nalc⁺X⁻-Rest, wobei X ein Halogenatom und alc ein Alkylrest mit bis zu 8 Kohlenstoffatomen ist, darstellt,
Y ein Wasserstoffatom, einen Hydroxylrest oder ein Halogenatom oder einen OSO₃H-Rest oder eines der Salze dieses Rests darstellt,
W ein Wasserstoffatom oder einen OH-Rest darstellt,
Z ein Wasserstoffatom oder einen Methylrest darstellt,
sowie die Additionssalze der Produkte der Formel (I) mit den Säuren.

2. Die in Anspruch 1 definierten Verbindungen der Formel (I), worin T ein Wasserstoffatom darstellt.

3. Die in Anspruch 1 oder 2 definierten Verbindungen der Formel (I), worin W ein Wasserstoffatom darstellt.

4. Die in einem der Ansprüche 1 bis 3 definierten Verbindungen der Formel (I), worin Z einen Methylrest darstellt.

5. Die in einem der Ansprüche 1 bis 4 definierten Verbindungen der Formel (I), worin Y ein Wasserstoffatom darstellt.

6. Die in einem der Ansprüche 1 bis 5 definierten Verbindungen der Formel (I), worin R₃ einen Methylrest darstellt.

7. Die in einem der Ansprüche 1 bis 6 definierten Verbindungen der Formel (I), worin R₄ einen Hydroxylrest darstellt.

8. Die in einem der Ansprüche 1 bis 7 definierten Verbindungen der Formel (I), worin R einen Rest 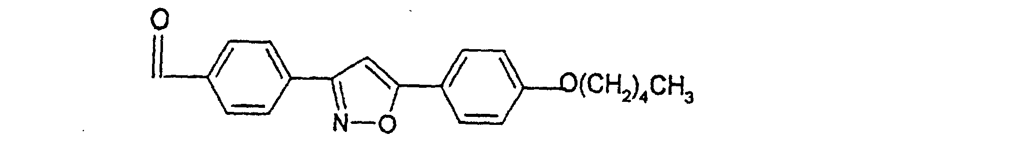 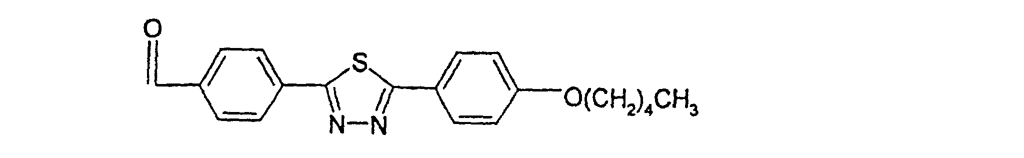 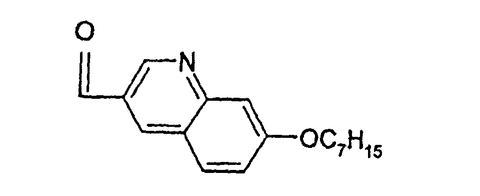 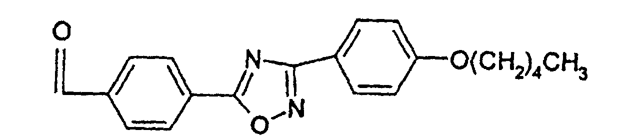 oder einen Rest 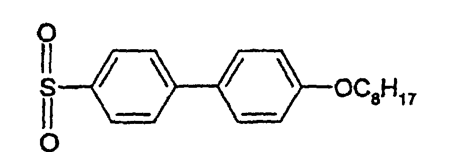 darstellt.

9. Die in einem der Ansprüche 1 bis 8 definierten Verbindungen der Formel I, worin R₁ einen Wasserstoffrest darstellt.

10. Die in einem der Ansprüche 1 bis 9 definierten Verbindungen der Formel I, worin R₂ einen Rest
(CH₂)₂NH₂
darstellt.

11. Die in einem der Ansprüche 1 bis 9 definierten Verbindungen der Formel I, worin R₂ einen Rest 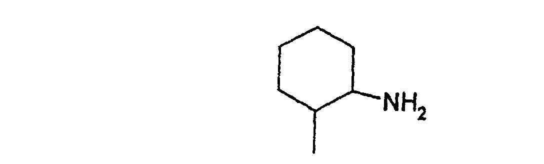 und insbesondere die Reste 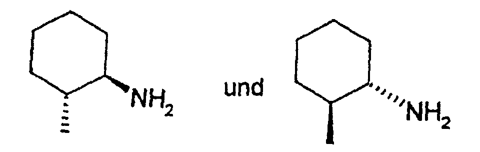 darstellt.

12. Die in einem der Ansprüche 1 bis 9 definierten Verbindungen der Formel I,
worin R₂ einen Rest 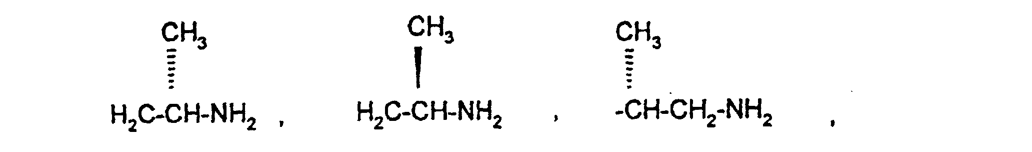 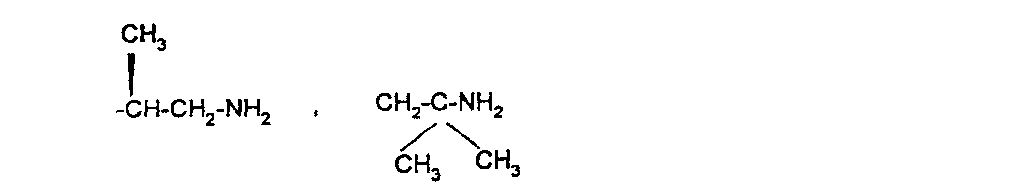 darstellt.

13. Die in Anspruch 1 definierten Verbindungen der Formel I, deren Namen folgen:
- 1-[4-[(2-Aminoethyl)-amino]-N2-[[4-[5-[4-(pentyloxy)-phenyl]-3-isoxazolyl]-phenyl]-carbonyl]-L-ornithin]-4-[4-(4-hydroxyphenyl)-L-threonin]-5-L-serinechinocandin B-trifluoracetat,
- trans-1-[4-[(2-Aminocyclohexyl)-amino]-N2-[[4-[5-(4-(pentyloxy)-phenyl]-3-isoxazolyl]-phenyl]-carbonyl]-L-ornithin]-4-[4-(4-hydroxyphenyl)-L-threonin]-5-Lserin-echinocandin B-trifluoracetat,
- 1-[4-[(2(S)-Aminopropyl)-amino]-N2-[[4-[5-[4-(pentyloxy)-phenyl]-3-isoxazolyl]-phenyl]-carbonyl]-L-ornithin]-4-[4-(4-hydroxyphenyl)-L-threonin]-5-L-serinechinocandin B-trifluoracetat,
- 1-[4-[(2-Aminoethyl)amino]-N2-[[4-[5-[4-(pentyloxy)-phenyl]-1,3,4-thiadiazol-2-yl]-phenyl]-carbonyl]-L-omithin]-4-[4-(4-hydroxyphenyl)-L-threonin]-5-L-serinechinocandin B-trifluoracetat,
- trans-1-[4-[(2-Aminocyclohexyl)-amino]-N2-[[4-[5-[4-(pentyloxy)-phenyl]-1,3,4-thiadiazol-2-yl]-phenyl]-carbonyl]-L-ornithin]-4-[4-(4-hydroxyphenyl)-L-threonin]-5-Lserin-echinocandin B-trifluoracetat,
- trans-1-[4-[(2-Aminocyclohexyl)-amino]-N2-[[4-[3-[4-(pentyloxy)-phenyl]-1,2,4-oxadiazol-5-yl]-phenyl]-carbonyl]-L-ornithin]-4-[4-(4-hydroxyphenyl)-L-threonin]-5-Lserin-echinocandin B-trifluoracetat.

14. Verfahren zur Herstellung der in einem der Ansprüche 1 bis 13 definierten Verbindungen der Formel (I), **dadurch gekennzeichnet, dass** man eine Verbindung der Formel (II) 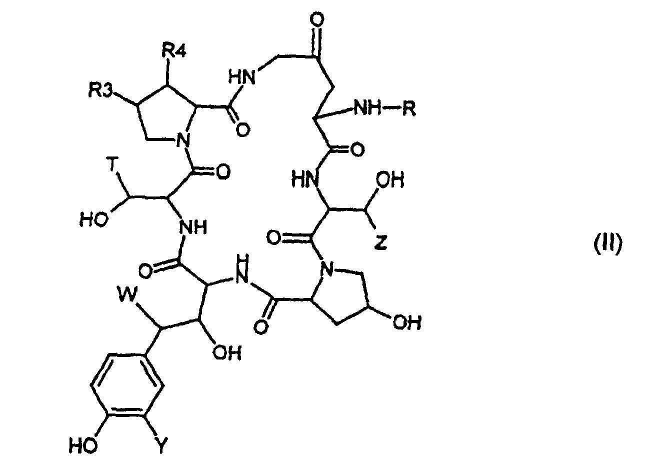 worin R, R₃, R₄, T, Y, W und Z die in den vorhergehenden Ansprüchen angegebene Bedeutung behalten, der Einwirkung eines Amins oder eines Aminderivats, das den 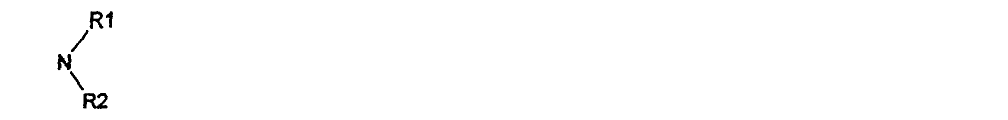 -Rest, worin R1 und R2 die in den vorhergehenden Ansprüchen angegebene Bedeutung behalten, einführen kann, und, wenn gewünscht, der Einwirkung eines Reduktionsmittels
und/oder eines Mittels zur Funktionalisierung des Amins,
und/oder einer Säure zur Bildung des Salzes des erhaltenen Produkts,
und/oder eines Mittels zur Trennung der verschiedenen erhaltenen Isomere unterzieht,
und so die Verbindung der Formel (I), wie in Anspruch 1 definiert, erhält.

15. Als neue chemische Produkte die in Anspruch 14 definierten Verbindungen der Formel (II).

16. Verfahren gemäß Anspruch 14, **dadurch gekennzeichnet, dass** man eine Verbindung der Formel (III) 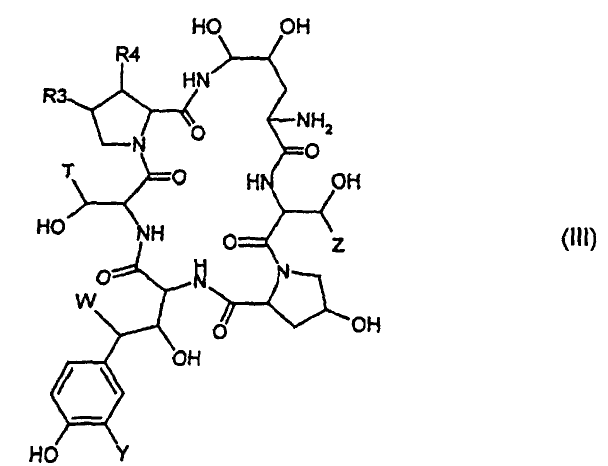 worin die verschiedenen Substituenten die in den vorhergehenden Ansprüchen angegebene Bedeutung behalten, der Einwirkung eines Mittels unterzieht, das NH₂ durch NHR, wobei R seine vorherige Bedeutung behält, ersetzen kann, um die Verbindung der Formel (IV) 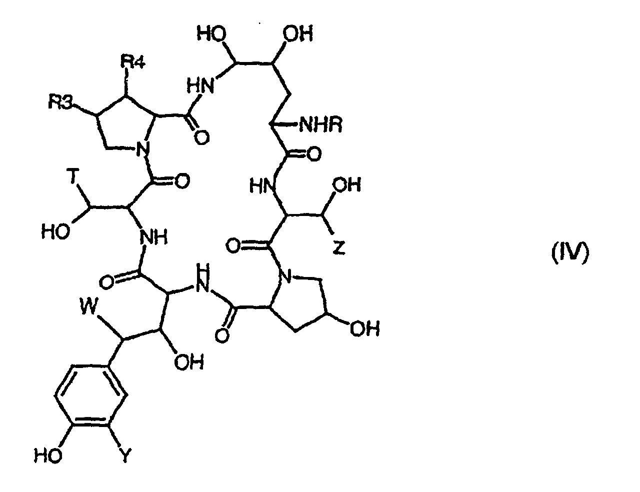 zu erhalten, die man der Einwirkung von Trimethylsilyliodid unterzieht, um die entsprechende Verbindung der Formel (II) 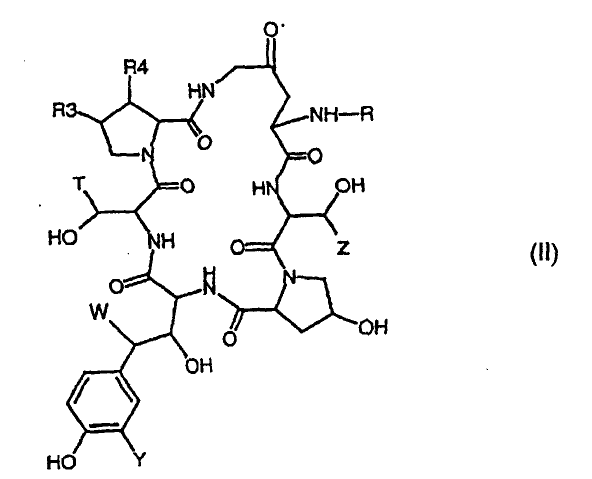 zu erhalten.

17. Als fungizide Verbindungen die in einem der Ansprüche 1 bis 13 definierten Verbindungen der Formel (I) sowie ihre Additionssalze mit den Säuren.

18. Die pharmazeutischen Zusammensetzungen, die als Medikament wenigstens eine in einem der Ansprüche 1 bis 13 definierte Verbindung der Formel (I) sowie ihre Additionssalze mit den pharmazeutisch annehmbaren Säuren enthalten.
